## Europäisches Patentamt
## European Patent Office
## Office européen des brevets

(19)

(11) Veröffentlichungsnummer: **0 287 003**
**B1**

(12) **EUROPÄISCHE PATENTSCHRIFT**

(45) Veröffentlichungstag der Patentschrift:
28.02.90

(51) Int. Cl.⁴: **A61K 35/78, A61K 7/48**

(21) Anmeldenummer: 88105692.3

(22) Anmeldetag: 09.04.88

(54) Verwendung von Biertreberextrakt zur Herstellung von medizinischen und kosmetischen Zubereitungen.

(30) Priorität: 16.04.87 DE 3712986

(43) Veröffentlichungstag der Anmeldung:
19.10.88 Patentblatt 88/42

(45) Bekanntmachung des Hinweises auf die Patenterteilung:
28.02.90 Patentblatt 90/9

(84) Benannte Vertragsstaaten:
AT BE CH DE ES FR GB GR IT LI LU NL SE

(56) Entgegenhaltungen:
FR-A- 2 379 282
US-A- 3 998 761

PARFUMS, COSMETIQUES, AROMES, Nr. 55,
Februar/März 1984, Seiten 47-54, Paris, FR; L. PEYRON:
"Le dioxyde de carbone liquide et supercritique"
CHEMICAL ABSTRACTS, Band 96, Nr. 24, 14. Juni 1982,
Seite 362, Zusammenfassung Nr. 205224e, Columbus,
Ohio, US; & JP-A-82 31 604 (OKASASU SHOTEN CO.,
LTD) 20-02-1982
SOVIET INVENTIONS ILLUSTRATED, Woche 8708, 4.
März 1987, Nr. 87-055591/08, Derwent Publications Ltd,
London, GB; & SU-A-1 242 190 (AEROZOL PRODN
COMBI) 07-07-1986

(73) Patentinhaber: Marbert GmbH, Bonner Strasse 155,
D-4000 Düsseldorf-Holthausen(DE)

(72) Erfinder: Motitschke, Lothar, Dr., Am Eichelkamp 18,
D-4010 Hilden(DE)
Erfinder: Tronnier, Hagen, Prof. Dr.,
Füssmannstrasse 12, D-4600 Dortmund 30(DE)

(74) Vertreter: Meier, Karl, Dr. et al, Hoechst AG Zentrale
Patentabteilung Postfach 80 03 20, D-6230 Frankfurt am
Main 80(DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die bei der Bierbrauerei nach dem Hopfenzusatz anfallende Restmaische ("Trub") wurde früher zur Bereitung medizinischer und kosmetischer Bäder, welche man als eine Art "Jungbrunnen" angesehen hat, verwendet; die Wirkung dieser Badezusätze wird auf aus dem Hopfen stammende östrogene zurückgeführt (Seifen-Öle-Fette-Wachse Nr.17/1961, Seite 530 und Nr.18/1961, Seite 555).

Aus einem anderen Nebenprodukt der Bierbrauerei, dem vor dem Hopfenzusatz anfallenden Biertreber, sollen desodorierende kosmetische Präparate erhältlich sein (FR-A 2 379 282). Dazu wird der Biertreber mit einem geeigneten Lösungsmittel - in der französischen Patentschrift sind Alkohole (Methanol, Ethanol, Butanol, Isopropanol), Ether, Ketone und Wasser (gegebenenfalls mit sauren oder basischen Zusätzen) genannt - extrahiert und der Extrakt konzentriert, gegebenenfalls vom Feststoff abfiltriert oder aber völlig von Lösungsmittel befreit. Aus dem aufkonzentrierten oder völlig vom Lösungsmittel befreiten Extrakt werden dann die entsprechenden kosmetischen Produkte (Lotionen, Cremes, Gels, Sprays etc.) auf übliche Weise hergestellt.

Wie die Nacharbeitung gezeigt hat, sind die nach der vorstehend genannten französischen Patentschrift erhaltenen Treberextrakte durchweg ziemlich dunkel (braun) gefärbt, was für die Herstellung weißer oder hell gefärbter Kosmetikpräparate nachteilig ist. Weiterhin besitzen diese Extrakte zwar die Eigenschaft, Schweiß und unangenehme Körpergerüche zu beseitigen oder zumindest zu vermindern, besitzen andererseits aber selbst einen zum Teil recht unangenehmen (stechenden) Eigengeruch. Als chromatographisch bestimmte Zusammensetzung des vom Lösungsmittel befreiten Extraktes ist in der französischen Patentschrift folgende Zusammensetzung angegeben:

Inhaltsstoffe eines ethanolischen Extraktes:

- Hordenin [4-(2-Dimethylaminoethyl)phenol]
- Aminosäuren (Alanin, Asparagin, Glutamin, Glutaminsäure, Isoleucin, Methionin, Phenylalanin, Prolin, Serin, Threonin, Tyrosin, Valin)
- Fettsäuren ($\alpha$-Aminobuttersäure, Citronensäure)
- Zucker (Fructose, Glucose, Saccharose, Raffinose)
- Elektrolyte.

Das Ausgangsprodukt für die Herstellung der medizinischen und kosmetischen Präparate - der sogenannte Biertreber - wird gemäß M. Becker und K. Nehring (Handbuch der Futtermittel, 3, S.102, Verlag P. Parey, Hamburg, Berlin, 1967) wie folgt erhalten:

Als Ausgangsstoffe können verschiedene Getreidesorten eingesetzt werden; vorzugsweise werden bestimmte Gerstensorten benutzt. Das Getreide wird vorzugsweise zunächst zur Einleitung des Keimprozesses einige Tage in Wasser geweicht. Anschließend erfolgt bei einer Temperatur von vorzugsweise ca.17 - 18°C die Keimung der Getreidekörner, wobei Enzyme - im wesentlichen - und $\beta$-Amylase - gebildet werden, die für die Umwandlung der Kohlenhydrate in vergärbare Zucker notwendig sind. Nach mehreren Tagen wird der Keimprozeß durch vorsichtiges Erhitzen und Trocknen der Körner abgebrochen. Das so entstandene Malz wird im nachfolgenden Arbeitsgang geschrotet, mit einer bestimmten Menge Wasser versetzt und auf eine Temperatur von vorzugsweise ca. 70 - 75°C erwärmt, was den Verzuckerungsprozeß einleitet. Die nach beendeter Verzuckerung aus der Suspension abgetrennten, unlöslichen Anteile werden als Treber bezeichnet, der bei der Bierbrauerei in großen Mengen anfällt. Eine Angabe über Zusammensetzung und Analyse der Biertreber findet sich in nachfolgender Tabelle aus "Mit Biertrebern mehr Ertrag und mehr Gewinn" (Informationsschrift Tremonis GmbH, Westfälische Str. 251, 4600 Dortmund 12.)

## Zusammensetzung der Biertreber

| | | Frisch von | bis | Siliert von | bis |
|---|---|---|---|---|---|
| Trockensubstanz TS (in %) | | 21,8 – | 24,7 | 23,6 – | 27,1 |
| Nährstoffe (in % der TS) | Rohprotein | 22,5 – | 27,5 | 22,3 – | 26,3 |
| | Rohfett | 5,5 – | 9,5 | 6,6 – | 10,8 |
| | Rohfaser | 16,2 – | 21,2 | 17,6 – | 22,4 |
| | Rohasche | 3,8 – | 6,2 | 3,7 – | 6,7 |
| | N-freie Extraktstoffe | 40,0 – | 47,0 | 37,3 – | 45,7 |
| | Stärke | | | 7,9 – | 9,2 |
| | Zucker | | | 1,3 – | 1,6 |
| Mineralstoffe (in % der TS) | Calcium | 0,27 – | 0,49 | 0,20 – | 0,46 |
| | Phosphor | 0,57 – | 0,77 | 0,43 – | 0,73 |
| | Natrium | 0,10 – | 0,90 | 0,23 – | 0,45 |
| | Magnesium | 0,17 – | 0,27 | 0,10 – | 0,34 |

| Spurenelemente (in mg je kg TS) | Eisen | 190 | Mangan | 50 | Kobalt | 0,18 |
|---|---|---|---|---|---|---|
| | Zink | 85 | Kupfer | 14 | Selen | 0,10 |

| Aminosäuren (in % der TS) | Lysin | 1,1 | Glutaminsäure | 4,3 | Leucin | 1,6 |
|---|---|---|---|---|---|---|
| | Methionin | 0,6 | Prolin | 2,0 | Thyrosin | 0,7 |
| | Cystin | 0,5 | Glycin | 0,9 | Phenylalanin | 0,1 |
| | Asparagin-säure | 1,4 | Alanin | 1,0 | Histidin | 0,5 |
| | Threonin | 0,9 | Valin | 1,3 | Arginin | 1,2 |
| | Serin | 0,8 | Isoleucin | 0,9 | Tryptophan | 0,2 |

| Vitamine (je kg TS) | Vitamin $B_1$ | 1,26 mg | Vitamin $B_{12}$ | 38,90 mcg | Ca-d-pant. | 3,03 mg |
|---|---|---|---|---|---|---|
| | Vitamin $B_2$ | 0,91 mg | Folsäure | 0,07 mg | Biotin | 140,00 mcg |
| | Vitamin $B_6$ | 0,62 mg | Nicotinsäure | 38,90 mg | Vitamin E | 25,70 mg |

| Fettsäuren (in % des Fettes) | C 8:0 | 0,1 | 16:0 (Palmitins.) | 21,7 | 20:0 | 0,8 |
|---|---|---|---|---|---|---|
| | 10:0 | – | 18:0 (Stearins.) | 5,0 | 22:0 | 2,9 |
| | 12:0 | 0,5 | 18:1 (Ölsäure) | 13,3 | 22:1 | 3,0 |
| | 14:0 | 1,3 | 18:2 (Linolensäure) | 27,5 | 24:0 | 1,3 |
| | | | 18:3 (Linolensäure) | 10,8 | Rest | 11,8 |

Es wurde nun gefunden, daß die wie vorstehend beschrieben gewonnenen Treberextrakte auch eine Heilwirkung für bestimmte, insbesondere mit Juckreiz und/oder entzündlichen Erscheinungen einhergehende, Hautkrankheiten besitzen und sich auch zur Herstellung von kosmetischen Präparaten ausgenommen Desodorantien, zur Pflege von trockener und/oder gereizter Haut und von trockener schuppiger Kopfhaut eignen.

Besonders vorteilhafte Treberextrakte sind für diese Zwecke solche, durch Extraktion von Biertreber mit flüssigem oder überkritischem $CO_2$ oder $N_2O$ erhaltene - nur schwach gefärbte (goldgelbe) und angenehm nach Getreide riechende - Extrakte. Dies ist außerordentlich überraschend, weil aufgrund der oben angegebenen Literaturstelle in der Zeitschrift Seifen-Öle-Fette-Wachse davon auszugehen war, daß nur solche Abfall oder Nebenprodukte der Bierbrauerei eine Heilwirkung besitzen, welche das aus dem Hopfen stammende Östrogen enthalten; vor dem Hopfenzusatz gewonnene Treber enthalten aber keine Östrogene, wie aus der vorstehenden Zusammensetzungsanalyse ersichtlich. Aus der in der französischen Patentschrift angegebenen Extraktzusammensetzung geht eindeutig hervor, daß sich derartige Östrogene auch nicht bei dem Extraktionsvorgang bilden.

Weiterhin konnte auch aufgrund der in der französischen Patentschrift beschriebenen desodorierenden Wirkung der entsprechenden Biertreberextrakte die völlig andersartige Eignung zur Behandlung und Pflege von zur Trockenheit neigender und/oder gereizter Haut und von trockener schuppiger Kopfhaut in keiner Weise erwartet werden, weil desodorierende Präparate üblicherweise in Körperregionen mit vermehrter Schweißbildung (Achselhöhlen etc.) - also auf feuchter Haut - aufgetragen werden.

Erfindungsgegenstand ist somit die Verwendung von Biertreberextrakt zur Herstellung von medizinischen Zubereitungen zur Behandlung von bestimmten, insbesondere mit Juckreiz und/oder entzündlichen Erscheinungen einhergehenden Hautkrankheiten sowie zur Herstellung von kosmetischen Präparaten, ausgenommen Desodorantien, zur Pflege von trockener und/oder gereizter Haut und von trockener schuppiger Kopfhaut. Der Biertreberextrakt kann mittels beliebigem Getreide (z.B. Gerste, Weizen, Hafer, Reis, Mais) hergestellt werden; vorzuziehen sind jedoch bestimmte Gerstensorten wie z.B. Hordeum distichum, Hordeum tetrastichum oder Hordeum hexastichum, insbesondere Hordeum distichum. Der Biertreber läßt sich naß oder trocken, vorzugsweise trocken extrahieren. Als Extraktionsmittel eignen sich im Prinzip alle in der vorerwähnten französischen Patentschrift angeführten Extraktionsmittel wie Alkohole, Ether, Ketone, Wasser, gegebenenfalls mit Zusatz von sauren oder basischen Stoffen. Darüber hinaus eignen sich auch andere Lösungsmittel wie z.B. Kohlenwasserstoffe (z.B. Petrolether, Hexan, Toluol) niedere halogenierte aliphatische Kohlenwasserstoffe (z.B. Dichlormethan, Trichlorfluormethan usw.), Ester (z.B. Ameisensäureethylester, Essigsäuremethylester, Essigsäureethylester usw.) und vorzugsweise sowohl $CO_2$ als auch $N_2O$ im flüssigen oder überkritischen Bereich; besonders bevorzugtes Lösungsmittel ist $CO_2$ im überkritischen Bereich.

Die Extraktion mit den herkömmlichen (unter Normalbedingungen flüssigen) Lösungsmitteln erfolgt auf an sich übliche Weise, wie z.B. auch in der französischen Patentschrift beschrieben. Die Extraktion mittels flüssigem oder überkritischem $CO_2$ erfolgt ebenfalls auf übliche Weise, wie z.B. in "Carbon dioxide extracted ingredients for fragrances" (Informationsschrift Pauls Flavours & Fragrances 0-5M385P, North Albert Road, Reigate, Surrey RH2 9ER, England) oder "Hochdruck-Extraktion" (Informationsschrift Hi 5 19200085 - HT80-5d+e+f Uhde GmbH, Buschmühlenstr. 20, 5800 Hagen 1) beschrieben; bevorzugte Parameter zur Treberextraktion mit überkritischem $CO_2$ sind Drucke von etwa 75 bis 600 bar, insbesondere zwischen etwa 150 und 350 bar und Temperaturen von etwa 31 bis 120°C, vorzugsweise von etwa 40 bis 90°C; bevorzugte Parameter zur Treberextraktion mit flüssigem $CO_2$ sind Drucke zwischen etwa 50 und 250 bar, insbesondere etwa 100 bis 150 bar, und Temperaturen zwischen etwa -10 bis 31°C, insbesondere zwischen etwa 10 und 20°C.

Die Extraktion mit flüssigem oder überkritischem $N_2O$ wird praktisch in der gleichen Weise und unter den gleichen Bedingungen durchgeführt, wobei lediglich die bevorzugte Temperaturgrenze für das Arbeiten mit dem flüssigen und dem überkritischen $N_2O$ geringfügig höher liegt - nämlich bei etwa 36,5°C - als bei der Extraktion mit $CO_2$ (etwa 31°C).

Die mittels herkömmlicher flüssiger Lösungsmittel gewonnenen Extrakte sind aufzukonzentrieren oder einzudampfen. Die mit flüssigem oder überkritischem $CO_2$ bzw. $N_2O$ gewonnenen Extrakte sind unter Normalbedingungen naturgemäß fast völlig frei von Extraktionsmittel. In der Zusammensetzung unterscheiden sich die einzelnen Extrakte laut dünnschichtchromatographischer Analyse kaum voneinander. Während die mittels herkömmlicher flüssiger Lösungsmittel gewonnenen Extrakte jedoch dunkel gefärbt sind und teilweise einen stechenden Geruch aufweisen, haben die $CO_2$- und $N_2O$-Extrakte eine goldgelbe Farbe und einen angenehmen, getreideartigen Geruch. Der Tropfpunkt der $CO_2$-Extrakte liegt zwischen etwa 35 und 39°C.

Die medizinischen Zusammensetzungen eignen sich als Mittel gegen Hautkrankheiten, vorzugsweise gegen Hautkrankheiten, die mit Juckreiz und/oder entzündlichen Erscheinungen einhergehen, insbesondere gegen Dermatitis atopica, Ichthyosis, Exsikkationsekzematoid, Pruritus, Sebostase und Dermatomykosen und Hautreizungen infolge von Quallenverbrennungen. Aufgrund der fehlenden vasokonstriktorischen Wirkung müssen andere Wirkungsmechanismen als bei Antihistaminika und Corticosteroiden vorliegen. Da Antihistaminika häufig nur eine geringe lokale Wirksamkeit und Corticosteroide die bekannten Nebenwirkungen zeigen, stellen die Biertreberextrakt enthaltenden medizinischen Zusammensetzungen eine therapeutische Alternative dar.

Die medizinischen Präparate auf der Grundlage der Biertreberextrakte sind vorzugsweise äußerlich anzuwenden.

Als Anwendungsform seien z.B. genannt: Lösungen, Suspensionen, Emulsionen, Pasten, Sälben, Gelees, Cremes, Lotionen, Puder, Seifen, tensidhaltige Reinigungspräparate, Öle und Sprays. Zusätzlich zu dem Biertreberextrakt werden der Zubereitung beliebige übliche Trägerstoffe, Hilfsstoffe und gegebenenfalls weitere Wirkstoffe zugesetzt. Vorzuziehende Hilfsstoffe stammen aus der Gruppe der Konservierungsstoffe, Antioxidantien, Stabilisatoren, Löslichmacher, Vitamine, Färbemittel, Geruchsverbesserer.

Salben, Pasten, Cremes und Gelees können neben dem oder den Wirkstoff(en) die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können neben dem oder den Wirkstoff(en) die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel z.B. Chlorfluorkohlenwasserstoffe, Propan/Butan oder Dimethylether enthalten.

4

Lösungen und Emulsionen können neben dem oder den Wirkstoff(en) die üblichen Trägerstoffe wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol, 1,3-Butylglykol, Öle, insbesondere Baumwollsaatöl, Erdnußöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können neben dem oder den Wirkstoff(en) die üblichen Trägerstoffe wie flüssige Verdünnungsmittel, z.B. Wasser, Ethanol, Propylenglykol, Suspendiermittel, z.B. ethoxylierte Isostearylalkohole, Polyoxyethylensorbit- und -sorbitanester, mikrokristalline Cellulose, Aluminiummetahydroxid, Bentonit, Agar-Agar und Traganth oder Gemische dieser Stoffe enthalten.

Seifen können neben dem oder den Wirkstoff(en) die üblichen Trägerstoffe wie z.B. Alkalisalze von Fettsäuren, Salze von Fettsäurehalbestern, Fettsäureeiweißhydrolysaten, Isethionate, Lanolin, Fettalkohol, Pflanzenöle, Pflanzenextrakte, Glycerin, Zucker oder Gemische dieser Stoffe enthalten.

Tensidhaltige Reinigungsprodukte können neben dem oder den Wirkstoff(en) die üblichen Trägerstoffe wie z.B. Salze von Fettalkoholsulfaten, Fettalkoholethersulfaten, Sulfobersteinsäurehalbestern, Fettsäureeiweißhydrolysaten, Isethionate, Imidazoliniumderivate, Methyllaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können neben dem oder den Wirkstoff(en) die üblichen Trägerstoffe, wie z.B. synthetische Öle wie Fettsäureester, Fettalkohole, Silikonöle, natürliche Öle wie Pflanzenöle und ölige Pflanzenauszüge, Paraffinöle, Lanolinöl oder Gemische dieser Stoffe enthalten.

Die Konzentration des Biertreberextraktes in den medizinischen Zubereitungen liegt zwischen etwa 0,5 und 100 Gew.-%, vorzugsweise zwischen etwa 5 und 99 Gew.-%. Die medizinische Zubereitung wird hergestellt, indem man den Biertreberextrakt und die jeweiligen Zusatzstoffe in an sich üblicher Weise in die jeweilig gewünschte Darreichungsform bringt.

Die Erfindung betrifft weiterhin die Verwendung von Biertreberextrakten zur Herstellung von kosmetischen Zubereitungen mit Ausnahme von Desodorantien zur Pflege von trockener und/oder gereizter Haut und von trockener schuppiger Kopfhaut. Da es für ein kosmetisches Erzeugnis besonders wichtig ist, einen angenehmen Geruch und eine ansprechende Färbung aufzuweisen, ist hier der $CO_2$- und der $N_2O$-Biertreberextrakt besonders wertvoll. Er kann, aufgrund seiner nur geringen Färbung und seines angenehmen Eigengeruchs, in höheren Konzentrationen als der herkömmliche Extrakt eingesetzt werden, ohne belästigend zu wirken.

Die Anwendungsformen der Kosmetikpräparate sind im Prinzip die gleichen wie für die medizinischen Zubereitungen und umfassen weiterhin auch typisch kosmetische Anwendungsformen wie Lippenstifte, Lippenpflegestifte, Mascara, Eyeliner, Rouge, Puder- und Emulsions-Make up etc. sowie Sonnenschutz- und After Sun-Präparate. Die Inhaltsstoffe für die kosmetischen Zubereitungen sind im wesentlichen die gleichen wie für die medizinischen Zubereitungen. Der Anteil an Biertreberextrakt beträgt vorzugsweise zwischen etwa 0,1 und 99 Gew.-%, insbesondere zwischen etwa 1 und 10 Gew.-%.

I. Untersuchung der therapeutischen Wirksamkeit


Test A: Histaminquaddeltest

Der Versuch wurde wie folgt durchgeführt:
Auf die vorgegebenen Meßflächen an der Haut des Rückens, mit einem Durchmesser von 7 cm wurden definierte Mengen der Prüfprodukte appliziert. Nach 15 Minuten Einwirkungszeit wurden die eingeriebenen Flächen mit einem Vlies abgedeckt und nach 2 Stunden Einwirkungszeit wurden in den Zentren der Felder Histaminquaddeln mit 0,02 ml einer 1/1000 %igen Histaminlösung gesetzt.

Nach 10, 20, 30, 40, 60 und 90 Minuten wurden die Erytheme und Quaddelgrößen in den Meßfeldern planimetrisch ausgewertet. Die Zahl der Probanden für diesen Test betrug 10. Es zeigte sich, daß alle geprüften Präparate gegenüber dem Kontrollfeld zu einer deutlichen Abschwächung der Reaktionen führten, was die antientzündliche Wirkung der eingesetzten Treberpräparate belegt.

Test B: Nikotinsäureestertest

Der Versuch wurde wie folgt durchgeführt:
Es wurden zunächst die Leerfelder mit einem Strahlungs-Thermometer sowie mit einem Laser-Doppler-Flowmeter ausgemessen. Anschließend wurden die Versuchspräparate aufgetragen.

Nach 2 Stunden wurden die vorbehandelten Felder sowie das Leerfeld erneut gemessen. Danach erfolgte pro Meßfeld eine Behandlung einschließlich des Leerfeldes mit 0,2 ml eines Nikotinsäureester-Präparates (®Rubriment-Öl, Basis Benzylnicotinat, Fa. Nordmark, Uetersen). Nach weiteren 30, 45, 60, 75, 90, 105 und 120 Minuten wurden dann Temperatur und Mikrozirkulation nochmals gemessen. Die Zahl der Probanden für diesen Test betrug 20.

Es zeigte sich, daß die Treberzubereitungen bei keinem der Probanden eine meßbare Beeinflussung der Nikotinsäureester-Reaktionen hatten. Die Treberzubereitungen haben demzufolge keine vasokonstriktorische Wirkung gegenüber einem Nikotinsäureester-Erythem.

Test C: Test der juckreizstillenden und antientzündlichen Wirkung

Bei 100 Patienten, 74 männliche und 26 weibliche, mit einem Durchschnittsalter von 32,5 Jahren wurden O/W(Öl in Wasser)- und W/O(Wasser in Öl)-Emulsionen mit 1 bis 5 %igem Treberölzusatz angewandt. Die Salben wurden 2mal täglich über 1 bis 4 Wochen aufgetragen. Die Verträglichkeit der angewandten Grundlagen war bei allen Patienten gut. Im Wirkungsvergleich, Halbseitenanwendung von Grundlage und Grundlage + Treberextrakt konnten bei 80 % der Probanden nach Anwendung der Treberzusammensetzung zusätzlich entzündungshemmende und juckreizstillende Effekte beobachtet werden. Die therapeutische Wirkung stellte sich schon nach wenigen Tagen Anwendung ein und konnte durch weitere Anwendung gesteigert werden.

Test D: Balneotherapeutischer Test

Bei insgesamt 230 Patienten wurden Treberextrakte in mehr als 2000 Badeanwendungen eingesetzt. Die Konzentration der Treberextrakte im Badewasser betrug 10 ml Extrakt pro 80 l Bad. Von den Patienten litten 50 an Psoriasis vulgaris, 150 an atopischer Dermatitis, 10 an urämischem Puritus, 10 an Prurigo und 10 an ekzema craquelé bzw. Exsikkationsekzematoid. Von nahezu allen Patienten wurde ein rückfettender Effekt des Bades beschrieben, die Pflegewirksamkeit wurde mit "gut" beurteilt. Die Patienten mit juckenden Dermatosen berichteten über eine Linderung des Juckreizes, die noch mehrere Stunden nach dem Bad anhielt.

Test E: Test der antimykotischen Wirkung

In einem in vitro- und einem in vivo-Versuch wurde die antimykotische Wirkung von Treberextrakt vergleichend untersucht. Im Therapiemodell bei der Meerschweinchen-Trichophytie (Erreger: Trichophyton mentagrophytes) wurde nach 5maliger Behandlung mit Treberextrakt ein antimykotischer Effekt erzielt, der identisch mit dem einer ®Batrafencreme-Zubereitung (Basis Ciclopiroxolamin, Fa. Cassella-Riedel, Frankfurt/Main) ist (siehe Tabelle 1).

Tabelle 1

| Therapiemodell Meerschweinchen-Trichophytie | | | |
|---|---|---|---|
| Präparat | Mykosedurchmesser x in mm | | x Bewertungszahl |
| Infektionskontrolle | 10, 11, 16 | 12,3 | |
| Treber-Extrakt | 7, 8, 7 | 7,3 | 50 |
| Batrafen | 3, 8, 11 | 7,3 | 50 |

II. Therapeutische Wirksamkeit im Vergleichsversuch

Bei einer großen Zahl von Patienten, die an Dermatitis atopica, Psoriasis, Exsikkationszuständen, Pruritus oder Sebostase litten und bereits Erfahrungen mit der Anwendung von marktüblichen Ölbädern, z.B. ®Balneum Hermal (Basis Sojaöl, Firma Hermal, Reinbek), ®Oleobal (Basis Sojaöl, Fa. Heilit, Reinbek) etc. hatten, wurden Treberextrakt-Bäder angewandt. Von fast allen Patienten wurde das Treberextrakt-Bad als sehr angenehm empfunden. Viele Patienten beschrieben einen rückfettenden Effekt, der ihnen auch von vorher angewandten Ölbädern bekannt war. Patienten mit juckenden Dermatosen, insbesondere Patienten mit atopischer Dermatitis (Patienten mit empfindlicher Haut) berichteten über eine Linderung des Juckreizes nach Anwendung des Bades. Aufgrund dieser positiven Erfahrung mit dem Treberbad, insbesondere bezüglich der juckreizstillenden Wirkung, zogen viele Patienten ein Treberbad dem früher angewandten Ölbad vor.

Die nachfolgenden Beispiele dienen der weiteren Erläuterung der Erfindung (alle Mengenangaben in Gewichtsprozent).

III. Medizinische Zubereitungen

1. Behandlungscreme

| | |
|---|---|
| $CO_2$-Treberextrakt | 10% |
| Propylenglykolmonodistearat | 15% |
| Konservierungsmittel | 0,01% |
| Wasser | ad 100% |

2. Gel

| | |
|---|---|
| $CO_2$-Treberextrakt | 15% |
| Carboxyvinylpolymere | 2% |
| Konservierungsmittel | 0,01% |
| Wasser | ad 100% |

3. Salbe

| | |
|---|---|
| $CO_2$-Treberextrakt | 20% |
| Pflanzenöl | 10% |
| Acetyllanolin | 10% |
| Lanolinalkohol | 12% |
| Sorbitansesquioleat | 2% |
| Wasser | ad 100% |

4. Alkohollotion

| | |
|---|---|
| $CO_2$-Treberextrakt | 25% |
| Oleylalkohol | 5% |
| Triethanolamin | 0,5% |
| Alkohol (95%) | ad 100% |

5. Zusatz für medizinisches Bad

| | |
|---|---|
| $CO_2$-Treberextrakt | 40% |
| Ammoniumlaurylsulfat | 10% |
| Laurylalkohol | 15% |
| Pflanzenöl | 15% |
| Lanolinwachs | 5% |
| Diisopropyladipat | 15% |

IV. Kosmetische Zubereitungen

### 1. W/O-Emulsion mit $CO_2$-Treberextrakt

| | |
|---|---|
| Glyceryl Sorbitan Isostearat | 8,0% |
| Methyl Glucose Dioleat | 2,0% |
| Talgglyceride | 2,0% |
| Lanolinalkohol | 2,0% |
| Paraffinöl | 8,0% |
| Isopropylstearat | 8,0% |
| Silikonöl | 2,0% |
| Pflanzenöle | 3,0% |
| Konservierungsmittel | 0,5% |
| Glycerin | 5,0% |
| Treberextrakt | 5,0% |
| Parfümöl | 0,5% |
| Wasser | ad 100,0% |

### 2. Cremebad mit $CO_2$-Treberextrakt

| | |
|---|---|
| Lanolinwachs | 5,0% |
| Diisopropyladipat | 13,0% |
| Paraffinöl | 5,0% |
| Pflanzenöl | 15,0% |
| Treberextrakt | 5,0% |
| Capryl-/Capringlyceride | 5,0% |
| Linolensäurediethanolamid | 5,0% |
| Parfümöl | 2,0% |
| Laurylalkohol | 15,0% |
| Laurylethersulfat | 30,0% |

### 3. O-/W-Emulsion mit $CO_2$-Treberextrakt

| | |
|---|---|
| Glycerinmonostearat selbstemulg. | 3,0% |
| Sorbitanstearat | 2,0% |
| Cetylalkohol | 3,0% |
| Lanolinalkohol | 5,0% |
| Treberextrakt | 3,0% |
| Isopropylstearat | 3,0% |
| Pflanzenöle | 7,0% |
| Silikonöle | 5,0% |
| Sorbitol | 5,0% |
| Polyacrylsäure | 0,2% |
| Triethanolamin | 0,3% |
| Konservierungsmittel | 0,4% |
| Wasser | ad 100,0% |

### 4. Massageöl mit $CO_2$-Treberextrakt

| | |
|---|---|
| Paraffinöl perliquidum | 48,3% |
| Octylpalmitat | 10,0% |
| Isopropylstearat | 13,0% |
| Silikonöle | 5,0% |
| Lanolinalkohol | 5,0% |
| Oleylalkohol | 5,0% |
| Pflanzenöle | 10,0% |
| Treberextrakt | 3,0% |
| Konservierungsmittel | 0,2 |
| Parfümöl | 0,5% |

### 5. Duschgel mit $CO_2$-Treberextrakt

| | |
|---|---|
| Magnesium Laurylethersulfat | 30,0% |
| Natrium Myristinethersulfat | 10,0% |
| Laurylethersulfat | 15,0% |
| Cocosfettsäure-Eiweißkondensat | 5,0% |
| hydriertes Rizinusöl | 2,0% |
| Treberextrakt | 3,0% |
| Parfümöl | 1,0% |
| Konservierungsmittel | 0,5% |
| Wasser | ad 100,0% |

### 6. Pflegespray mit $CO_2$-Treberextrakt

| | |
|---|---|
| Silikonöle | 10,0% |
| hydriertes Rizinusöl | 2,0% |
| Teberextrakt | 3,0% |
| Ethylalkohol | 82,0% |
| Vitamin F-Ethylester | 2,0% |
| Menthol | 0,5% |
| Parfümöl | 0,5% |

### Abfüllung:

| | |
|---|---|
| Wirkstoff | 83 g |
| [R] Frigen 12 | 43 g |
| ($CCl_2F_2$, Fa. Hoechst AG, Frankfurt/Main) | |
| [R] Frigen 114 ($CClF_2CClF_2$, | 28 g |
| Fa. Hoechst AG, Frankfurt/Main) | |

## Patentansprüche

1. Verwendung von Biertreberextrakt zur Herstellung von medizinischen Zubereitungen zur Behandlung von Hautkrankheiten, welche insbesondere mit Juckreiz und/oder entzündlichen Erscheinungen einhergehen.

2. Verwendung von Biertreberextrakt nach Anspruch 1, dadurch gekennzeichnet, daß die zu behandelnden Hautkrankheiten Dermatitis atopica, Ichthyosis, Exsikkationsekzematoid, Pruritus, Sebostase, Dermatomykosen und Quallenverbrennungen sind.

3. Verwendung von Biertreberextrakt nach Anspruch 1 oder 2, dadurch gekennzeichnet, daß der Biertreberextrakt mittels flüssigem oder überkritischem CO₂ oder N₂O gewonnen wurde.

4. Verwendung von Biertreberextrakt nach einem oder mehreren der Ansprüche 1 bis 3, dadurch gekennzeichnet, daß die medizinischen Zubereitungen solche zur äußeren Anwendung in Form einer Lösung, einer Suspension, einer Emulsion, einer Paste, einer Salbe, eines Gelees, einer Creme, einer Lotion, eines Puders, einer Seife, eines tensidhaltigen Reinigungsproduktes, eines Öls und eines Sprays sind.

5. Verwendung von Biertreberextrakt zur Herstellung von kosmetischen Zubereitungen mit Ausnahme von Desodorantien.

6. Verwendung von Biertreberextrakt nach Anspruch 5, dadurch gekennzeichnet, daß die kosmetischen Zubereitungen zur Pflege von trockener und/oder gereizter Haut und von trockener, schuppiger Kopfhaut bestimmt sind.

7. Verwendung von Biertreberextrakt nach Anspruch 5 oder 6, dadurch gekennzeichnet, daß der Biertreberextrakt mittels flüssigem oder überkritischem CO₂ oder N₂O gewonnen wurde.

## Claims

1. The use of brewers' grains extract for the preparation of medicinal compositions for the treatment of skin disorders which are particularly associated with pruritus and/or inflammatory manifestations.

2. The use of brewers' grains extract as claimed in claim 1, wherein the skin disorders to be treated are atopic dermatitis, ichthyosis, preeczematous states, pruritus, sebostasis, mycoses of the skin and jellyfish stings.

3. The use of brewers' grains extract as claimed in claim 1 or 2, wherein the brewers' grains extract has been obtained using liquid or above-critical $CO_2$ or $N_2O$.

4. The use of brewers' grains extract as claimed in one or more of claims 1 to 3, wherein the medicinal compositions are those for external use in the form of a solution, a suspension, an emulsion, a paste, an ointment, a gel, a cream, a lotion, a dusting powder, a soap, a surfactant-containing cleansing product, an oil and a spray.

5. The use of brewers' grains extract for the preparation of cosmetic compositions with the exception of deodorants.

6. The use of brewers' grains extract as claimed in claim 5, wherein the cosmetic compositions are intended for care of dry and/or irritated skin and of dry scalp with dandruff.

7. The use of brewers' grains extract as claimed in claim 5 or 6, wherein the brewers' grains extract has been obtained using liquid or above-critical $CO_2$ or $N_2O$.

## Revendications

1. Utilisation d'extrait de drêche de bière pour la fabrication de préparations médicinales pour le traitement d'affections cutanées, en particulier accompagnées de prurit ou de phénomènes inflammatoires.

2. Utilisation d'extrait de drêche de bière conformément à la revendication 1, caractérisée en ce que les affections cutanées à traiter sont la dermatite atopique, l'ichthyosis, l'eczéma desséchant, le prurit, la sébostase et les dermatomycoses et les brûlures dues aux méduses.

3. Utilisation d'extrait de drêche de bière conformément à la revendication 1 ou 2, caractérisée en ce que l'on obtient l'extrait de drêche de bière au moyen de $CO_2$ ou $N_2O$ liquides ou supercritiques.

4. Utilisation d'extrait de drêche de bière conformément à l'une quelconque des revendications 1 à 3, caractérisée en ce que les préparations médicinales sont celles pour une application externe sous forme d'une solution, d'une suspension, d'une émulsion, d'une pâte, d'un onguent, d'une gelée, d'une crème, d'une lotion, d'une poudre, d'un savon, d'une composition détergente tensioactive, d'une huile et d'un spray.

5. Utilisation d'extrait de drêche de bière pour la fabrication de préparations cosmétiques à l'êxception des désodorisants.

6. Utilisation d'extrait de drêche de bière conformément à la revendication 5, caractérisée en ce que les préparations cosmétiques servent au soin des peaux sèches et/ou irritées et du cuir chevelu sec squaneux.

7. Utilisation d'extrait de drêche de bière conformément à la revendication 5 ou 6, caractérisée en ce que l'on obtient l'extrait de drêche de bière au moyen de $CO_2$ ou $N_2O$ liquides ou supercritiques.